# EUROPEAN PATENT SPECIFICATION

(11) **EP 3 191 025 B1**
(45) Date of publication and mention of the grant of the patent: **09.12.2020**
(21) Application number: 15757527.5
(22) Date of filing: 08.09.2015
(51) Int. Cl.: A61F 2/24

(54) **ANNULOPLASTY IMPLANT**
ANNULOPLASTIE-IMPLANTAT
IMPLANT D'ANNULOPLASTIE

(30) Priority: 08.09.2014 US 201462047077 P
(43) Date of publication of application: 19.07.2017
(73) Proprietor: Medtentia International Ltd Oy, 02600 Espoo (FI)
(72) Inventor: O'CARROLL, Ger, Co. Sligo (IE); PUGH, Mark, Co. Sligo, Coolaney (IE); MORAN, Adrian, Co. Sligo (IE); ZERKOWSKI, Hans-Reinhard, CH-4125 Reihen (CH); KERÄNEN, Olli, S-237 41 Bjärred (SE)
(86) International application number: PCT/EP2015/070464
(87) International publication number: WO 2016/038017

(56) References cited:
- EP-A1- 3 090 703
- WO-A1-2006/091163
- CN-A- 103 735 337
- US-A- 5 628 790
- US-A1- 2005 049 698
- US-A1- 2005 256 569
- US-A1- 2008 077 235
- US-A1- 2010 121 433
- US-A1- 2011 060 401

## Description

### Field of the Invention

This invention pertains in general to the field of cardiac valve replacement and repair. More particularly the invention relates to an annuloplasty implant, such as an annuloplasty ring or helix, for positioning at the heart valve annulus.

### Background of the Invention

Diseased mitral and tricuspid valves frequently need replacement or repair. The mitral and tricuspid valve leaflets or supporting chordae may degenerate and weaken or the annulus may dilate leading to valve leak. Mitral and tricuspid valve replacement and repair are frequently performed with aid of an annuloplasty ring, used to reduce the diameter of the annulus, or modify the geometry of the annulus in any other way, or aid as a generally supporting structure during the valve replacement or repair procedure.

Annuloplasty rings devised for implantation are over time overgrown and encapsulated by tissue. The process of endothelialization, leading to the encapsulation of the implant by tissue, depends on the surface properties of the implant. Incomplete or delayed endothelialization can be a cause of embolism or thrombosis in a later stage after implantation.

A problem with prior art annuloplasty implants is the compromise between the functionality of the implant during the initial stages, such as during the implantation procedure, and the long term characteristics of the implant, for example with respect to the endothelialization process.

A further problem of prior art devices is the lack of flexibility of the implant in certain situations, which impedes optimal functioning when implanted in the moving heart, or adaptability to varying anatomies.

An annuloplasty implant is intended to function for years and years, so it is critical with long term stability. Material fatigue may nevertheless lead to rupture of the material, that may be unexpected and uncontrolled. This entails a higher risk to the patient and it is thus a further problem of prior art devices.

The above problems may have dire consequences for the patient and the health care system. Patient risk is increased.

Hence, an improved annuloplasty implant would be advantageous and in particular allowing for improved properties during the initial implantation phase, and long term functioning.

US 2010/121433 A1 discloses in the embodiment of Fig. 18 an annuloplasty implant comprising an inner core of a shape memory material and an outer cloth covering formed of a thin strip of material wound helically around the inner core.

### Summary of the Invention

Accordingly, embodiments of the present invention preferably seeks to mitigate, alleviate or eliminate one or more deficiencies, disadvantages or issues in the art, such as the above-identified, singly or in any combination by providing a device according to the appended patent claims.

According to a first aspect an annuloplasty implant is provided comprising an inner core of a shape memory material, an outer covering arranged radially outside said inner core material to cover at least part of said inner core, wherein said outer covering is resilient to conform to said inner core during movement of said shape memory material, wherein said outer covering comprises a first material having surface properties to promote endothelialization.

According to a second aspect an annuloplasty implant is provided comprising a shape memory material, a recess along a portion of said implant to reduce the cross-sectional area thereof at said recess, wherein two portions of said implant are joined at said recess and are flexible with respect to eachother by a bending motion at said recess.

Further embodiments of the invention are defined in the dependent claims, wherein features for the second and subsequent aspects of the invention are as for the first aspect mutatis mutandis.

Some embodiments of the invention provide for improved endothealialization.

Some embodiments of the invention provide for prevention of late embolism or thrombosis.

Some embodiments of the invention provides for increased safety in case of material fatigue and rupture.

Some embodiments of the invention provide for a more flexible implant.

Some embodiments of the invention provide for a low-profile implant.

Some embodiments of the invention provide for facilitated delivery of the implant to the target site.

Some embodiments of the invention provide for minimized friction of the implant against the delivery catheter.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1a is an illustration of an annuloplasty implant according to an embodiment of the invention;
Fig. 1b is an illustration of the annuloplasty implant in Fig. 1a in a cross-sectional view, according to an embodiment of the invention;
Fig. 2 is an illustration of an annuloplasty implant according to an embodiment of the invention in a detail view from Fig. 1b;
Fig. 3 is an illustration of an annuloplasty implant in a detail view from Fig. 1b;
Fig. 4 is an illustration of an annuloplasty implant, in a helix or coil shape, according to an embodiment of the invention;
Fig. 5 is an illustration of an annuloplasty implant according to an embodiment of the invention;
Fig. 6 is an illustration of an annuloplasty implant, in a detailed view, according to an embodiment of the invention;
Fig. 7 is an illustration of an annuloplasty implant in a perspective view according to an embodiment of the invention;

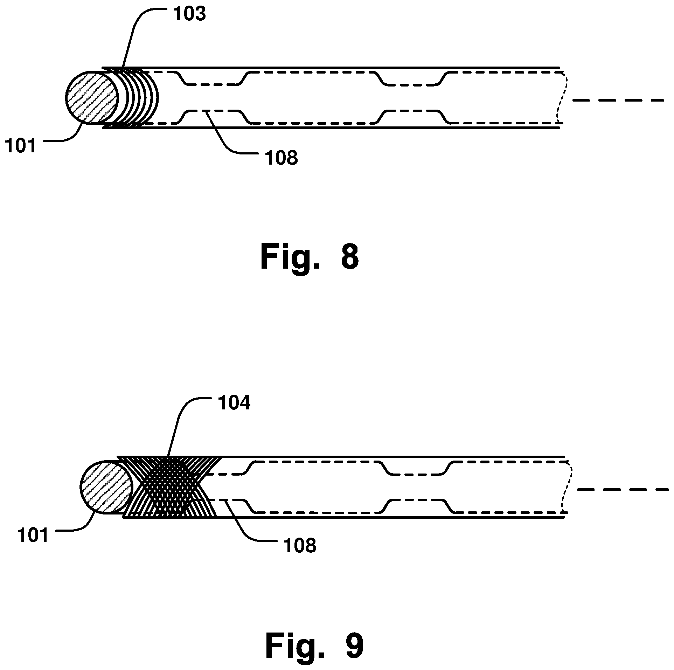

Fig. 8 is an illustration of an annuloplasty implant, in a detailed view, according to an embodiment of the invention; and
Fig. 9 is an illustration of an annuloplasty implant, in a detailed view.

### Description of embodiments

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The following description focuses on an embodiment of the present invention applicable to cardiac valve implants such as annuloplasty rings. However, it will be appreciated that the invention is not limited to this application but may be applied to many other annuloplasty implants and cardiac valve implants including for example replacement valves, and other medical implantable devices.

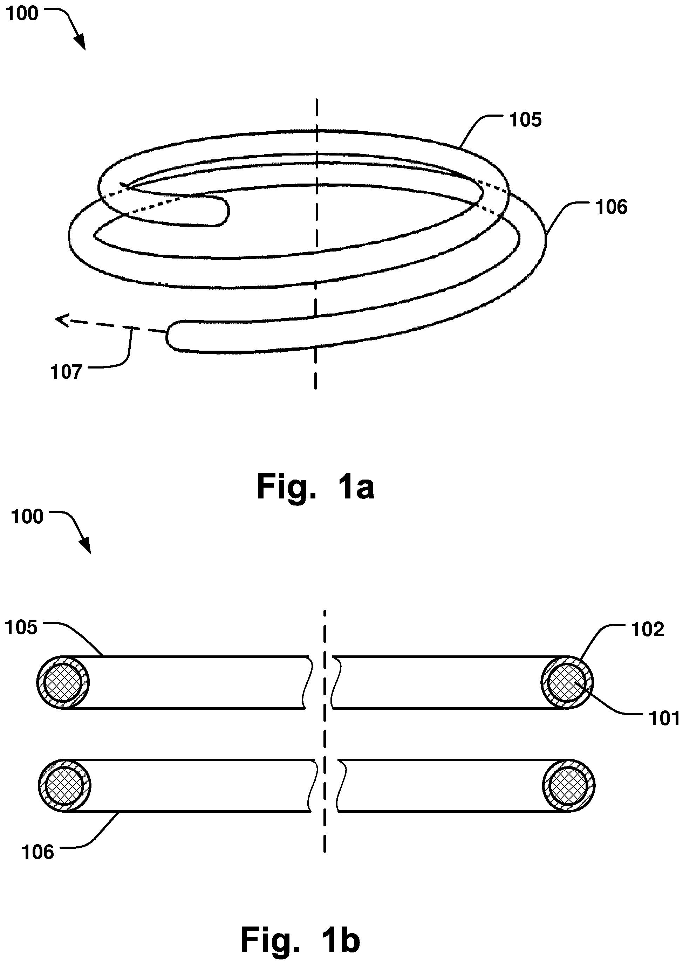

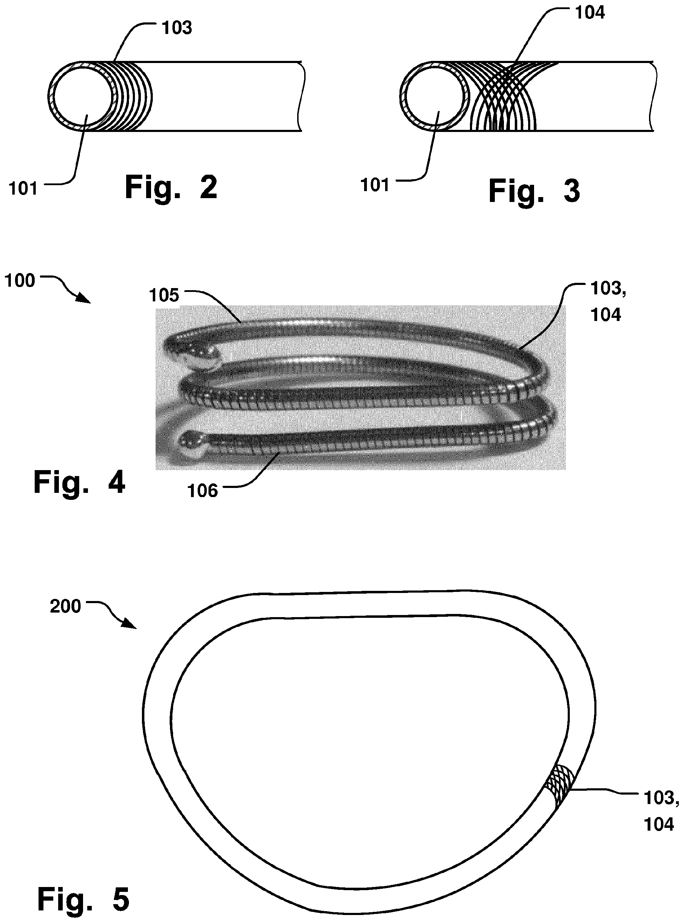

Fig. 1a shows an annuloplasty implant 100 comprising an inner core 101 of a shape memory material, an outer covering 102 arranged radially outside said inner core material to cover at least part of said inner core, wherein the outer covering is resilient to conform to the inner core during movement of the shape memory material. Thus, the outer covering readily follows the movement of the inner core material, such when stretching the ring during delivery in a catheter, and subsequently, returning to the ring shape after released from the confinement of the catheter. The outer covering comprises a first material having surface properties to promote endothelialization. The first material may be a metal alloy. The material of the inner core material can thus be optimized for providing the desired shape memory properties, such as fast recovery to the predefined implanted shape, while the outer covering 102 is customized to promote the endothelialization process. This dual functionality removes the issue of having to compromise between the most desired shape memory properties in the initial stage and the long term characteristics desired to accelerate endothelialization and minimize the risk of late embolism. The latter functionality thus needs not to be dictated by the core material, which may not provide for the most desired surface characteristics for endothelialization. Similarly, the shape memory effect would have been impeded if the annuloplasty implant would have been tailored for endothelialization purely. Thus a synergetic effect is obtained by having such customized core material and covering. The core material may comprise a single wire or filament. The core material may also comprise a plurality of wires or filaments. The number of wires or filaments may be varied as desired in order to provide the desired properties of the core material, such as the desired flexibility, shape memory effects, or cross-sectional dimension, that is preferred for the procedure. The annuloplasty implant may be both a helix or coil shape as seen in Fig. 1a and 4, or a closed ring 200 as seen in Fig. 5. Any type of ring, such as open ring or C-shaped ring is also possible. Fig. 1b shows a cross-section of the implant 100 for illustrating the inner core 101 and the outer covering 102.

The outer covering 102 comprises a spiral 103 wound around the inner core 101, as illustrated in the detailed view of Fig. 2, and in Fig. 4. The spiral may easily conform to the shape of the core material and follow movement thereof without affecting shape memory properties. The spiral can provide for an increased surface roughness that ease the formation of endothelia cells over the surface and reduces the time for the endothelialization process and tissue overgrowth. At the same time, the surface of the outer covering is sufficiently smooth, with a low friction coefficient, so that the implant slides into place easily and minimizes any interference with the tissue. As can be seen in Fig. 4, the coil comprises a wire having a flattened cross-sectional profile, that can provide such smooth surface.

In an example not part of the present invention, the outer covering may comprise a mesh or braiding 104 of strands, as illustrated in the detailed view of Fig. 3. The mesh may also easily conform to the shape of the core material and follow movement thereof without affecting shape memory properties. The mesh can provide for an increased surface roughness that ease the formation of endothelia cells over the surface and reduces the time for the endothelialization process and tissue overgrowth. The implant 100, 200 may have any combination of spirals and mesh on different portions of the implant. Fig. 5 illustrates just a portion of the implant 200 having a covering 103, 104, for sake of clarity of presentation only.

The outer covering 102 may have a predefined surface porosity or roughness to start endothealialization within a set time period. Thus it is possible to customize the surface properties to attain the desired endothealialization process, to minimize embolism.

The outer covering 102 may cover substantially the entire core 101 in the longitudinal direction 107 of the implant 100, 200. This may provide for optimized endothealialization across the entire length of the implant. The covering may also have different properties on different parts of the implant 100, 200. In case having a coil shaped ring the ring placed towards the atrium or the ventricle may have different properties than the other ring.

The implant may comprise a catheter deliverable ring 100, wherein said ring has an elongated delivery configuration for advancement in a catheter and an implanted shape assuming a predefined configuration of said shape memory material for positioning at a heart valve annulus. Thus the ring in the implanted shape may comprise a first 105 and second 106 support members arranged in a coiled configuration, and being adapted to be arranged on opposite sides of native heart valve leaflets to pinch said leaflets.

The outer covering may cover the first and second support members. Alternatively, the covering may only be provided at one of the rings, or have different properties for the rings as mentioned above.

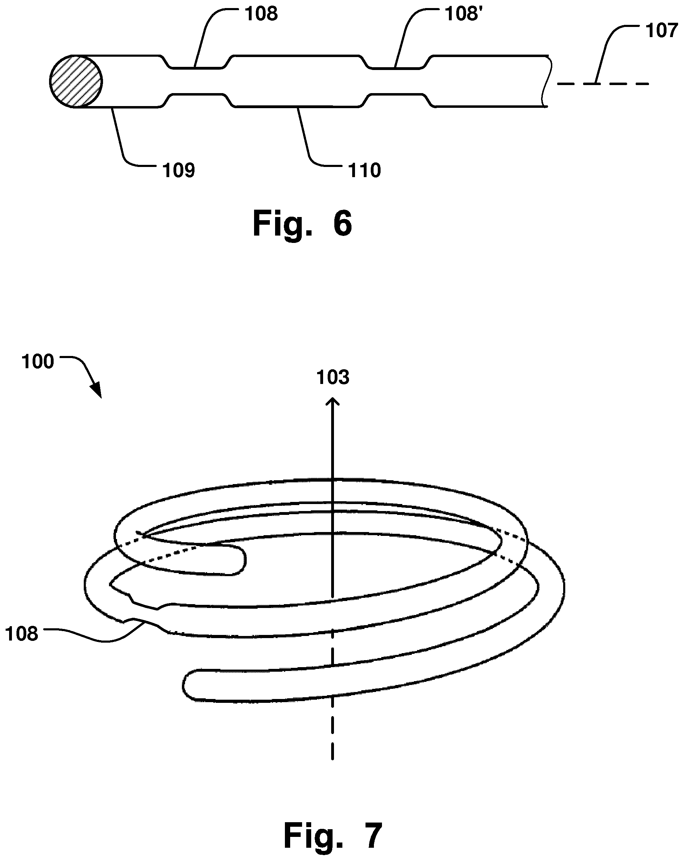

The annuloplasty implant 100, 200, may comprise a recess 108 along a portion of the implant to reduce the cross-sectional area thereof at said recess, as illustrated in Figs. 6 and 7.

Two portions 109, 110 of the implant 100, 200 may thus be joined at said recess 108 and be flexible with respect to eachother by a bending motion at the recess 108. The recess can thus serve to increase the flexibility of the implant, at defined locations where more movement is desired. The recess is provided in the inner core 101 of the shape memory material.

The two portions 109, 110, may also have a predefined breaking point at the recess 108. Thus since the amount of material is less at the reduced cross-section of the implant it is possible to define preferred breaking points of the implant, to avoid random breaking in case of material fatigue occurs after a long time. The location of the breaking point can thus be positioned to not cause any damage to the patient. Further, even if the core material is not broken, the material properties may change over time, e.g. becoming less flexible due to material hardening, and the recess will thus still provide flexibility to the implant.

The annuloplasty implant may comprise a plurality of said recesses 108, 108', along a longitudinal direction 107 of said implant, as seen in Fig. 6. A plurality of flexing or breaking points may thus be provided as desired where flexing, or possibly breaking, is preferred.

The covering may be arranged over said recess. In case of having a covering 102, there will also be an additional increase in safety since the covering will prevent any broken parts to be dislodged into the patient.

The first material may comprise a first metal alloy that is bio compatible, such as stainless steel, NiTinol, or any other metal alloy that is suitable for formation of endothelia. In addition of the advantageous properties of such metal alloy for the endothelialization process, the metal alloy covering over the inner core provides for reduced friction against a delivery catheter, compared to e.g. surfaces being more porous and/or having higher friction coefficients such as textile coverings. It is also conceivable to have a polymer covering that also has a very low friction coefficient, similar to that of the surface of a metal alloy. This may thus facilitate delivery of the implant, and allowing a more controlled delivery, since the implant moves more easily through the delivery catheter. It is thus possible to optimize the outer covering for providing advantageous formation of endothelia, while at the same time reducing friction, and further having the inner core optimized for the desired shape-memory properties as described above. The effect of having reduced friction can also be advantageously combined with having the recess 108, 108', in the core material, providing for the advantageous effects as described above with respect to the recess 108, 108'.

Having a metal alloy as outer covering provides also for a compact implant with a minimized cross-sectional dimension, while allowing for the optimization of the shape memory properties of the core material simultaneous as having the optimized properties of the covering with respect to endothelialization, as well as the low-friction properties described in the foregoing. The compact cross-sectional dimension allows for using a thinner catheter, that can be advantageous in some procedures, and/or facilitates the simultaneous use of additional instruments that can be inserted in parallel lumens of the catheter during the procedure.

The covering may comprise any polymer and is not limited to a metal alloy.

The inner core may comprise a second material such as a second metal alloy, different from said first material or first metal alloy, such as NiTinol, or any other alloy that provides for the desired shape memory effect. The inner core may comprise any polymer and is not limited to a metal alloy. Both metal alloys and polymers can be treated during manufacturing to have a desired heat-set shape, which is the shape the implant strives towards when any restraining force is removed, i.e. the relaxed shape, such as when the implant is pushed out of the delivery catheter which forces the implant into an elongated shape. It is also possible that the implant assumes the desired implanted shape by activation of the shape memory function of the material, such as by addition of energy, e.g. heating, electromagnetic energy etc, or by mechanical restructuring of the material.

It is also disclosed an annuloplasty implant without a covering according to one example not part of the invention. Such annuloplasty implant comprises a shape memory material and a recess 108 along a portion of said implant to reduce the cross-sectional area thereof at said recess, wherein two portions 109, 110 of said implant are joined at said recess and are flexible with respect to eachother by a bending motion at said recess. This provides for the above mentioned advantages.

The present invention has been described above with reference to specific embodiments. However, other embodiments than the above described are equally possible within the scope of the invention. The different features and steps of the invention may be combined in other combinations than those described. The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. An annuloplasty implant (100, 200) comprising
an inner core (101) of a shape memory material,
an outer covering (102) arranged radially outside said inner core material to cover at least part of said inner core, wherein said outer covering is resilient to conform to said inner core during movement of said shape memory material, wherein said outer covering is formed of a first material having surface properties to promote endothelialization, wherein said outer covering is a spiral (103) wound around said inner core, **characterized in that** said spiral is formed of a wire having a flattened cross-sectional profile.

2. Annuloplasty implant according to claim 1, wherein said outer covering comprises a mesh or braiding (104) of strands.

3. Annuloplasty implant according to any of claims 1-2, wherein said implant comprises a catheter deliverable ring (100), wherein said ring has an elongated delivery configuration for advancement in a catheter and an implanted shape assuming a predefined configuration of said shape memory material for positioning at a heart valve annulus.

4. Annuloplasty implant according to claim 3, wherein said ring in the implanted shape comprises a first (105) and second (106) support members arranged in a coiled configuration, and being adapted to be arranged on opposite sides of native heart valve leaflets to pinch said leaflets.

5. Annuloplasty implant according to claim 4, wherein said outer covering covers substantially the entire core in a longitudinal direction (107) of the implant.

6. Annuloplasty implant according to claim 4, wherein said outer covering covers the first and second support members.

7. Annuloplasty implant according to any of claims 1-6, comprising a recess (108) along a portion of said implant to reduce the cross-sectional area thereof at said recess.

8. Annuloplasty implant according to claim 7, wherein two portions (109, 110) of said implant are joined at said recess and are flexible with respect to eachother by a bending motion at said recess.

9. Annuloplasty implant according to claim 8, wherein said two portions has a predefined breaking point at said recess.

10. Annuloplasty implant according to any of claims 7-9, comprising a plurality of said recesses (108,108') along a longitudinal direction (107) of said implant.

11. Annuloplasty implant according to any of claims 6-10, wherein said covering is arranged over said recess.

12. Annuloplasty implant according to any of claims 1-11, wherein said first material comprises a first metal alloy.

13. Annuloplasty implant according to any of claims 1-12, wherein said inner core comprises a second material, different from said first material, and wherein said second material comprises a second metal alloy such as NiTinol.

## Patentansprüche

1. Anuloplastie-Implantat (100, 200), umfassend einen inneren Kern (101) aus einem Formgedächtnismaterial,
eine äußere Abdeckung (102), die radial außerhalb des inneren Kernmaterials angeordnet ist, um mindestens einen Teil des inneren Kerns abzudecken, wobei die äußere Abdeckung elastisch ist, um sich während einer Bewegung des Formgedächtnismaterials an den inneren Kern anzupassen, wobei die äußere Abdeckung aus einem ersten Material mit Oberflächeneigenschaften gebildet ist, um Endothelialisierung zu fördern, wobei die äußere Abdeckung eine um den inneren Kern herumgewickelte Spirale (103), **dadurch gekennzeichnet, dass** die Spirale aus einem Draht mit einem abgeflachten Querschnittsprofil gebildet ist.

2. Anuloplastie-Implantat nach Anspruch 1, wobei die äußere Abdeckung ein Netz oder ein Geflecht (104) aus Strängen umfasst.

3. Anuloplastie-Implantat nach einem der Ansprüche 1 - 2, wobei das Implantat einen mittels eines Katheters zuführbaren Ring (100) umfasst, wobei der Ring eine längliche Zuführkonfiguration zum Vorschieben in einem Katheter und eine implantierte Form hat, die eine vordefinierte Konfiguration des Formgedächtnismaterials zur Positionierung an einem Herzklappenanulus annimmt.

4. Anuloplastie-Implantat nach Anspruch 3, wobei der Ring in der implantierten Form ein erstes (105) und ein zweites (106) Stützglied umfasst, die in einer aufgespulten Konfiguration angeordnet und dazu ausgeführt sind, an gegenüberliegenden Seiten von nativen Herzklappensegeln angeordnet zu werden, um die Segel zu quetschen.

5. Anuloplastie-Implantat nach Anspruch 4, wobei die äußere Abdeckung im Wesentlichen den gesamten Kern in einer Längsrichtung (107) des Implantats abdeckt.

6. Anuloplastie-Implantat nach Anspruch 4, wobei die äußere Abdeckung das erste und das zweite Stützglied abdeckt.

7. Anuloplastie-Implantat nach einem der Ansprüche 1 - 6, umfassend eine Aussparung (108) entlang eines Abschnitts des Implantats, um dessen Querschnittsfläche an der Aussparung zu reduzieren.

8. Anuloplastie-Implantat nach Anspruch 7, wobei zwei Abschnitte (109, 110) des Implantats an der Aussparung miteinander verbunden sind und durch eine Biegebewegung an der Aussparung bezüglich einander flexibel sind.

9. Anuloplastie-Implantat nach Anspruch 8, wobei die beiden Abschnitte eine Sollbruchstelle an der Aussparung haben.

10. Anuloplastie-Implantat nach einem der Ansprüche 7 - 9, umfassend eine Vielzahl der Aussparungen (108, 108') entlang einer Längsrichtung (107) des Implantats.

11. Anuloplastie-Implantat nach einem der Ansprüche 6 - 10, wobei die Abdeckung über der Aussparung angeordnet ist.

12. Anuloplastie-Implantat nach einem der Ansprüche 1 - 11, wobei das erste Material eine erste Metalllegierung umfasst.

13. Anuloplastie-Implantat nach einem der Ansprüche 1 - 12, wobei der innere Kern ein sich von dem ersten Material unterscheidendes zweites Material umfasst und wobei das zweite Material eine zweite Metalllegierung wie NiTinol umfasst.

## Revendications

1. Implant d'annuloplastie (100, 200), comprenant :
un noyau intérieur (101) en matériau à mémoire de forme,
un recouvrement extérieur (102) disposé radialement à l'extérieur dudit matériau de noyau intérieur pour recouvrir au moins une partie dudit noyau intérieur, ledit recouvrement extérieur étant élastique de manière à épouser la forme dudit noyau intérieur au cours du mouvement dudit matériau à mémoire de forme, ledit recouvrement extérieur étant formé d'un premier matériau ayant des propriétés de surface favorisant l'endothélialisation, ledit recouvrement extérieur étant une spirale (103) enroulée autour dudit noyau intérieur, **caractérisé en ce que** ladite spirale est formée d'un fil ayant un profil aplati en section transversale.

2. Implant d'annuloplastie selon la revendication 1, dans lequel ledit recouvrement extérieur comprend un maillage ou un tressage (104) de brins.

3. Implant d'annuloplastie selon l'une quelconque des revendications 1 et 2, ledit implant comprenant un anneau pouvant être introduit par cathéter (100), ledit anneau ayant une configuration d'introduction allongée prévue pour lui permettre d'avancer dans un cathéter et une forme implantée adoptant une configuration prédéfinie dudit matériau à mémoire de forme pour le positionnement au niveau d'un anneau de valvule cardiaque.

4. Implant d'annuloplastie selon la revendication 3, dans lequel ledit anneau, dans la forme implantée, comprend un premier (105) et un deuxième (106) organe de support disposés dans une configuration enroulée et étant adaptés pour être disposés sur des côtés opposés de feuillets de valvule cardiaque natifs pour pincer lesdits feuillets.

5. Implant d'annuloplastie selon la revendication 4, dans lequel ledit recouvrement extérieur recouvre sensiblement la totalité du noyau dans une direction longitudinale (107) de l'implant.

6. Implant d'annuloplastie selon la revendication 4, dans lequel ledit recouvrement extérieur recouvre les premier et deuxième organes de support.

7. Implant d'annuloplastie selon l'une quelconque des revendications 1 à 6, comprenant un renfoncement (108) le long d'une portion dudit implant pour réduire la surface en section transversale de celui-ci au niveau dudit renfoncement.

8. Implant d'annuloplastie selon la revendication 7, dans lequel deux portions (109, 110) dudit implant sont réunies au niveau dudit renfoncement et peuvent fléchir l'une par rapport à l'autre par un mouvement de flexion au niveau dudit renfoncement.

9. Implant d'annuloplastie selon la revendication 8, dans lequel lesdites deux portions présentent un point de rupture prédéfini au niveau dudit renfoncement.

10. Implant d'annuloplastie selon l'une quelconque des revendications 7 à 9, comprenant une pluralité desdits renfoncements (108, 108') le long d'une direction longitudinale (107) dudit implant.

11. Implant d'annuloplastie selon l'une quelconque des revendications 6 à 10, dans lequel ledit recouvrement est disposé par-dessus ledit renfoncement.

12. Implant d'annuloplastie selon l'une quelconque des revendications 1 à 11, dans lequel ledit premier matériau comprend un premier alliage métallique.

13. Implant d'annuloplastie selon l'une quelconque des revendications 1 à 12, dans lequel ledit noyau intérieur comprend un deuxième matériau différent dudit premier matériau et dans lequel ledit deuxième matériau comprend un deuxième alliage métallique tel que du nitinol.
